# EUROPÄISCHE PATENTSCHRIFT

(11) **EP 2 384 317 B1**
(45) Veröffentlichungstag und Bekanntmachung des Hinweises auf die Patenterteilung: **23.04.2014**
(21) Anmeldenummer: 09796682.4
(22) Anmeldetag: 14.12.2009
(51) Int. Cl.: C07C 45/28, C07C 49/04

(54) **VERFAHREN ZUR HERSTELLUNG VON KETONEN DURCH UMSETZUNG VON 1,1-DISUBTITUIERTEN OLEFINEN MIT N2O**
METHOD FOR PRODUCING KETONES BY CONVERTING 1.1-DISUBSTITUTED OLEFINES BY MEANS OF N2O
PROCÉDÉ DE PRODUCTION DE CÉTONES PAR RÉACTION D'OLÉFINES 1,1-DISUBSTITUÉES AVEC DU N2O

(30) Priorität: 30.12.2008 EP 08173047
(43) Veröffentlichungstag der Anmeldung: 09.11.2011
(73) Patentinhaber: BASF SE, 67056 Ludwigshafen (DE)
(72) Erfinder: TELES, Joaquim Henrique, 67166 Otterstadt (DE); SCHELPER, Michael, 67065 Ludwigshafen (DE)
(86) Internationale Anmeldenummer: PCT/EP2009/067121
(87) Internationale Veröffentlichungsnummer: WO 2010/076182

(56) Entgegenhaltungen:
- US-A1- 2006 106 258
- STAROKON E V ET AL: "LIQUID PHASE OXIDATION OF ALKENES WITH NITROUS OXIDE TO CARBONYL COMPOUNDS" ADVANCED SYNTHESIS & CATALYSIS, WILEY VCH VERLAG, WEINHEIM, DE LNKD- DOI:10.1002/ADSC.200303155, Bd. 346, Nr. 2-3, 1. Januar 2004 (2004-01-01), Seiten 268-274, XP002321384 ISSN: 1615-4150 in der Anmeldung erwähnt

## Beschreibung

Die vorliegende Erfindung betrifft ein Verfahren zur Herstellung eines Ketons umfassend die Umsetzung einer Zusammensetzung (I) mindestens enthaltend ein 1,1-disubstituiertes Olefin mit einer Zusammensetzung enthaltend Distickstoffmonoxid, wobei die Umsetzung in Gegenwart eines Lösungsmittels erfolgt, das mindestens eine protonenliefernde funktionelle Gruppe enthält.

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. In beiden Schriften wird allgemein offenbart, dass die Oxidation prinzipiell in Anwesenheit eines geeigneten Oxidationskatalysators erfolgen kann.

GB 649,680 beschreibt die Oxidation von Olefinen mit Distickstoffmonoxid, wobei als Produkt ein Gemisch aus einem Keton und einem Cyclopropanderivat gewonnen wird. Es wird weiter erwähnt, dass diese zwei Produktverbindungen durch fraktionierende Destillation voneinander getrennt werden können. Nachteilig ist aber, dass ein Produktgemisch erhalten wird, das durch eine aufwändige Reinigung getrennt werden muss.

In den neueren wissenschaftlichen Artikeln von G. I. Panov et al., "Non-Catalytic Liquid Phase Oxidation of Olefines with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Olefines with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Olefines with Nitrous Oxide to Carbonyl Compounds" von E. V. Starokon et al. in Adv. Synth. Catal. 2004, 346, 268 - 274 beinhaltet eine mechanistische Studie der Oxidation von Olefinen mit Distickstoffmonoxid in flüssiger Phase.

Die Synthese von Carbonylverbindungen aus Olefinen mit Distickstoffmonoxid wird auch in verschiedenen internationalen Patentanmeldungen beschrieben. So offenbart die WO 03/078370 ein Verfahren zur Herstellung von Carbonylverbindungen aus α-liphatischen Olefinen mit Distickstoffmonoxid. Die Umsetzung wird bei Temperaturen im Bereich von 20 bis 350°C und Drücken von 1,01 bis 10132,5 kPa (0,01 bis 100 atm) durchgeführt. Die WO 03/078374 offenbart ein entsprechendes Verfahren zur Herstellung von Cyclohexanon. Gemäß der WO 03/078372 werden cyclische Ketone mit 4 bis 5 C-Atomen hergestellt. Gemäß der WO 03/078375 werden unter diesen Verfahrensbedingungen cyclische Ketone aus cyclischen Olefinen mit 7 bis 20 C-Atomen hergestellt. WO 03/078371 offenbart ein Verfahren zur Herstellung substituierter Ketone aus substituierten Olefinen. WO 04/000777 offenbart ein Verfahren zur Umsetzung von Di- und Polyolefinen mit Distickstoffmonoxid zu den entsprechenden Carbonylverbindungen.

WO 03/78370 beschreibt die Oxidation von Olefinen im Allgemeinen mit N₂O unter Bildung von Ketonen mit der gleiche C-Zahl. Insbesondere wird dort die Oxidation von 2-Penten ohne Lösungsmittel und in Anwesenheit von verschiedenen Lösungsmitteln (Toluol, Mesitylen, Cyclohexan, Cyclohexanon, Acetonitril und Isobutanol) beschrieben. Die Versuche zeigen, dass weitgehend identische Ergebnisse erzielt werden, unabhängig davon, welches Lösungsmittel eingesetzt wird.

In WO 2005/030690 und WO 2005/030689 werden Verfahren zur Herstellung von Cyclododecanon beschrieben, wobei in einem Verfahrensschritt eine Oxidation mit Distickstoffmonoxid erfolgt. In WO 2005/030690 wird ein Verfahren zur Herstellung von Cyclododecanon durch Oxidation von 1,5,9-Cyclododecatrien (CDT) mit N₂O zu Cyclo-dodeca-4,8-dienon und die anschließende Hydrierung von Cyclododeca-4,8-dienon zu Cyclododecanon beschrieben.

Allen Verfahren gemeinsam ist, dass die Reinheit der Rohprodukte ohne zusätzliche Reinigung für einige Anwendungen nicht ausreicht. Insbesondere Cyclopropanderivate sind häufig in hohen Mengen in den erhaltenen Produkten enthalten.

Dies ist insofern problematisch, als Ketone für verschiedene Anwendungen in hoher Reinheit benötigt werden. Deshalb ist in diesen Fällen eine sehr aufwändige Reinigung, beispielsweise durch vielstufige Destillation und/oder Kristallisation, notwendig. Diese Reinigungsverfahren sind dadurch aufwändig und kostenintensiv.

Der vorliegenden Erfindung lag daher die Aufgabe zu Grunde, ein Verfahren bereit zu stellen, mit dem Ketone einfach und mit geringem Aufwand in hoher Reinheit erhalten werden können.

Erfindungsgemäß wird diese Aufgabe gelöst durch ein Verfahren zur Herstellung eines Ketons der allgemeinen Formel (1) 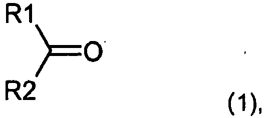 worin R1 und R2 unabhängig voneinander ausgewählt sind aus substituierten und unsubstituierten Alkylresten mit 1 bis 20 Kohlenstoffatomen, und substituierten und unsubstituierten Arylresten, und wobei die Reste R1 und R2 auch miteinander verbunden sein und einen Ring bilden können,
umfassend die Umsetzung einer Zusammensetzung (I) mindestens enthaltend ein 1,1-disubstituiertes Olefin der allgemeinen Formel (2) 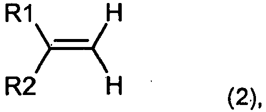 mit einer Zusammensetzung enthaltend Distickstoffmonoxid, wobei die Umsetzung in Gegenwart eines Lösungsmittels erfolgt, das mindestens eine protonenliefernde funktionelle Gruppe enthält und aus der Gruppe der Lösungsmittel, die mindestens eine COOH-Gruppe aufweisen, der Lösungsmittel, die mindestens eine OH-Gruppe aufweisen, und der Lösungsmittel, die mindestens eine NH-Gruppe aufweisen, ausgewählt ist.

Durch das erfindungsgemäße Verfahren können Ketone mit hoher Reinheit, beispielsweise einer Reinheit von > 99,5 %, erhalten werden. Das erfindungsgemäße Verfahren

ist sehr selektiv, so dass keine weiteren Reinigungsschritte nötig sind und weniger Produkt verloren geht.

Erfindungsgemäß können als Lösungsmittel alle Substanzen eingesetzt werden, die unter den Reaktionsbedingungen nicht mit N₂O reagieren und eine protonenliefernde funktionelle Gruppe enthalten. Geeignet sind Lösungsmittel, die mindestens eine COOH-Gruppe aufweisen, Lösungsmittel, die mindestens eine OH-Gruppe aufweise, oder Lösungsmittel, die mindestens eine NH-Gruppe aufweisen.

Erfindungsgemäß geeignete Beispiele für Verbindungen mit einer COOH-Gruppe sind insbesondere Essigsäure, verzweigte und unverzweigte substituierte oder unsubstituierte Carbonsäuren mit einer oder mehreren Carboxylgruppen, mit bis zu 20 Kohlenstoffatomen, aromatische Carbonsäuren, sowie Aminosäuren.

Erfindungsgemäß geeignete Beispiele für Verbindungen mit einer OH-Gruppe sind Wasser, aliphatische Alkohole mit 1 bis 20 Kohlenstoffatomen, aliphatische Diole mit 2 bis 20 Kohlenstoffatomen, aliphatische Triole mit 3 bis 20 Kohlenstoffatomen, aliphatische Polyole mit 4 oder mehr OH-Gruppen und 4 bis 20 Kohlenstoffatomen, Phenol, sowie substituierte Phenole.

Erfindungsgemäß geeignete Beispiele für Verbindungen mit einer NH-Gruppe sind Ammoniak, Hydrazin, primäre und sekundäre aliphatische Amine mit 1 bis 40 Kohlenstoffatomen, aliphatische Di- und Polyamine mit 2 bis 20 Kohlenstoffatomen, aromatische Amine, sowie primäre und sekundäre Amide.

Gemäß einer weiteren Ausführungsform betrifft die vorliegende Erfindung daher ein Verfahren zur Herstellung eines Ketons wie oben beschrieben, wobei das Lösungsmittel ausgewählt ist aus der Gruppe bestehend aus Carbonsäuren, Aminosäuren, Wasser, Alkoholen, Ammoniak, Hydrazin, Aminen und Amiden.

Erfindungsgemäß geeignete aliphatische Alkohole sind beispielsweise Methanol, Ethanol, 1-Propanol, 2-Propanol, 1-Butanol, 2-Butanol, Isobutanol, tert-Butanol, 1-Pentanol, 2-Pentanol, Isopentanol, tert-Pentanol, Cyclopentanol, 3-Methyl-2-butanol, 1-Hexanol, 2-Hexanol, 3-Hexanol, 2-Methylpentanol-1, Cyclohexanol, 1-Heptanol, 1-Octanol, 2-Octanol, 2-Ethylhexanol, 1-Nonanol, N-Nonanol, 1-Decanol, 2-Propylheptanol, 2-Propyl-3-methyl-1-pentanol, 1-Undecanol, 1-Dodecanol, 2-Butyloctanol, 2-Butyl-3-methyl-1-heptanol, 1-Tridecanol, N-Tridecanol, 1-Tetradecanol, 1-Hexadecanol, 1-Octadecanol, 1-Icosanol, 2-Methoxyethanol, 2-Ethoxyethanol, 2-Butoxyethanol, 2-Phenoxyethanol, Diethylenglykolmonomethylether, Triethylenglykolmonomethylether, Tetraethylenglykolmonomethylether, 2-Methoxy-1-propanol, 1-Methoxy-2-propanol, Dipropylenglykolmonomethylether, Tripropylenglykolmonomethylether, 4-Methoxy-1-butanol, 5-Methoxy-1-pentanol, 6-Methoxy-1-hexanol, und Glycerincarbonat.

Erfindungsgemäß geeignete aliphatische Diole sind beispielsweise Ethylenglykol, 1,2-Propandiol, 1,3-Propandiol, 1,2-Butandiol, 2,3-Butandiol, 1,4-Butandiol, 1,2-Pentandiol, 1,5-Pentandiol, 1,6-Hexandiol, Neopentylglykol, 1,8-Octandiol, 1,10-Dodecandiol, 1,11-Undecandiol, 1,12-Dodecandiol, 1,13-Tridecandiol, 1,14-Tetradecandion, 1,18-Octadecandiol, Diethylenglykol, Triethylenglykol, Tetraethylenglykol, Dipropylenglykol, und Tripropylenglykol.

Erfindungsgemäß geeignete aliphatische Triole sind beispielsweise Glycerin, 1,2,4-Butantriol, Trimethylolethan, Trimethylolpropan, Trimethylolbutan, Diglycerin, 2-[2,2-Bis-(2-hydroxy-ethoxymethyl)-butoxy]-ethanol, Triethanolamin, und Tripropanolamin.

Erfindungsgemäß geeignete aliphatische Polyole mit mindestens 4 C-Atomen sind beispielsweise 1,2,3,4-Butantetrol, Pentaerithrit, Sorbitol, und 2-[3-(2-Hydroxy-ethoxy)-2,2-bis-(2-hydroxy-ethoxymethyl)-propoxy]-ethanol.

Erfindungsgemäß geeignete Phenole sind beispielsweise Phenol, p-Kresol, o-Kresol, m-Kresol, 2,6-Xylenol, 2,4-Xylenol, 2,5-Xylenol, 2,3,6-Trimethylphenol, Mesitol, o-Chlorphenol, m-Chlorphenol, p-Chlorphenol, 2-Methoxyphenol, 4-Methoxyphenol, alpha-Naphtol, und beta-Naphtol.

Erfindungsgemäß geeignete Carbonsäuren sind beispielsweise Essigsäure, Propionsäure, Buttersäure, Isobuttersäure, Pivalinsäure, Valeriansäure, 2-Methylbuttersäure, Isovaleriansäure, Hexansäure, 2-Methylpentansäure, Heptansäure, Octansäure, 2-Ethylhexansäure, 2-Propylheptansäure, Isononansäure, Neodecansäure, Stearinsäure, Benzoesäure, und o-Tolylsäure.

Erfindungsgemäß geeignete primäre und sekundäre aliphatische Amine mit 1 bis 40 Kohlenstoffatomen sind beispielsweise Methylamin, Ethylamin, Propylamin, Isopropy-lamin, Butylamin, Isobutylamin, sec-Butylamin, tert-Butylamin, Pentylamin, Hexylamin, Heptylamin, Octylamin, 2-Ethylhexylamin, 3,5,5-Trimethylhexylamin, 2-Propylheptylamin, Dimethylamin, Diethylamin, Dipropylamin, Diisopropylamin, Dibutylamin, Diisobutylamin, Di-sec.-butylamin, Dipentylamin, Dihexylamin, Diheptylamin, Dioctylamin, Di-(2-ethylhexyl)amin, Di-(3,5,5-Trimethylhexyl)amin, Di-(2-Propylheptyl)amin, Pyrrolydin, Piperidin, und Morpholin.

Erfindungsgemäß geeignete aliphatische Di- und Polyamine mit 2 bis 20 Kohlenstoffatome sind beispielsweise 1,2-Ethylendiamin, 1,3-Diaminopropan, N,N-Dimethyl-1,3-diaminopropan, Bis-(3-Dimethylaminopropyl)-amin, Bis-(2-Aminoethyl)-amin, 1,4-Diaminobutan, 1,5-Diaminopentan, 1,6-Diaminohexan, und Piperidin.

Erfindungsgemäß geeignete aromatische Amine sind beispielsweise Anilin, N-Methylanilin, o-Toluidin, m-Toluidin, p-Toluidin, und 2,6-Xylilendiamin.

Erfindungsgemäß geeignete primäre und sekundäre Amide sind beispielsweise Formamid, Acetamid, N-Methylacetamid, Pyrrolidon, Caprolactam, und Lauryllactam.

Als Lösungsmittel können erfindungsgemäß auch Verbindungen eingesetzt werden, die gleichzeitig mindestens eine OH- und mindestens eine NH-Gruppe enthalten. Erfindungsgemäß geeignet sind beispielsweise Ethanolamin, 1-Amino-2-propanol, 1-Amino-2-methyl-2-propanol, 2-Amino-2-methyl-1-propanol, 2-Amino-2-methyl-1,3-propandiol, 2-Amino-2-hydroxymethyl-1,3-propandiol, Diethanolamin, oder Dipropanolamin.

Es können ebenfalls Gemische aus zwei oder mehr der genannten Verbindungen eingesetzt werden.

Erfindungsgemäß wird eine Zusammensetzung (I) mindestens enthaltend ein 1,1-disubstituiertes Olefin mit einer Zusammensetzung enthaltend Distickstoffmonoxid umgesetzt wobei die Zusammensetzung (I) mindestens ein 1,1-disubstituierten Olefin der allgemeinen Formel (2) enthält 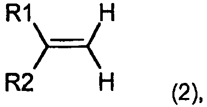 wobei R1 und R2 unabhängig voneinander ausgewählt sind aus substituierten und unsubstituierten Alkylresten mit 1 bis 20 Kohlenstoffatomen, und substituierten und unsubstituierten Arylresten, und wobei die Reste R1 und R2 auch miteinander verbunden sein und einen Ring bilden können.

Besonders bevorzugt sind das 1,1-disubstituierte Olefin ein Olefin der allgemeinen Formel (2) ist und die Reste R1 und R2 ausgewählt aus substituierten und unsubstituierten Alkylresten mit 1 bis 6 Kohlenstoffatomen und substituierten und unsubstituierten Arylresten.

Gemäß einer weiteren Ausführungsform betrifft die vorliegende Erfindung daher ein Verfahren zur Herstellung eines Ketons wie oben beschrieben, wobei das 1,1-disubstituierte Olefin ein Olefin der allgemeinen Formel (2) ist und die Reste R1 und R2 ausgewählt sind aus substituierten und unsubstituierten Alkylresten mit 1 bis 6 Kohlenstoffatomen und substituierten und unsubstituierten Arylresten.

Erfindungsgemäß geeignet sind beispielsweise Isobuten, Methylencyclopropan, 2-Methyl-1-buten, 2,4,4-Trimethyl-1-penten, 2,2,6,6-Tetramethyl-4-methylen-heptan, alpha-Polyisobuten, Methylencyclopentan, Methylencyclohexan, beta-Pinen und 1,1-Diphenylethen.

Gemäß einer weiteren Ausführungsform betrifft die vorliegende Erfindung daher ein Verfahren zur Herstellung eines Ketons wie oben beschrieben, wobei das 1,1-disubstituierte Olefin ausgewählt ist aus der Gruppe bestehend aus Isobuten, Methy-lencyclopropan, 2-Methyl-1-buten, 2,4,4-Trimethyl-1-penten, 2,2,6,6-Tetramethyl-4-methylen-heptan, alpha-Polyisobuten, Methylencyclopentan, Methylencyclohexan, beta-Pinen und 1,1-Diphenylethen.

Erfindungsgemäß enthält die Zusammensetzung (I) das 1,1-disubstituierte Olefin üblicherweise in einer Menge von mehr als 80 Gew.-%, vorzugsweise 85 bis 99,999 Gew.-%, insbesondere 90 bis 99,99 Gew.-%, besonders bevorzugt 92 bis 99,9 Gew.-%, beispielsweise 95 bis 99,8 Gew.-%. Die Zusammensetzung (I) kann neben dem 1,1-disubstituierte Olefin üblicherweise weitere Verbindungen, insbesondere organische Verbindungen, enthalten.

Die Zusammensetzung (I) mindestens enthaltend ein 1,1-disubstituiertes Olefin der allgemeinen Formel (2) wird mit einer Zusammensetzung enthaltend Distickstoffmonoxid umgesetzt. Im Rahmen der vorliegenden Erfindung kann Distickstoffmonoxid in reiner Form oder in Form eines Gasgemisches enthaltend Distickstoffmonoxid eingesetzt werden.

Grundsätzlich kann in dem erfindungsgemäßen Verfahren jedes Gasgemisch enthaltend Distickstoffmonoxid eingesetzt werden. Erfindungsgemäß ist es auch möglich, das Gasgemisch enthaltend Distickstoffmonoxid vor Einsatz in die Umsetzung zu reinigen oder aufzukonzentrieren. Ein geeignetes Reinigungsverfahren umfasst beispielsweise die Absorption des Gasgemisches in einem organischen Lösungsmittel oder Wasser, die Desorption des Gasgemisches aus dem beladenen organischen Lösungsmittel oder dem beladenen Wasser und das Einstellen des Gehalts an Stickoxiden NOx in dem Gasgemisch auf höchstens 0,01 bis 0,001 Vol.-%" bezogen auf das Gesamtvolumen des Gasgemisches. Ein derartiges Verfahren ist beispielsweise in DE 10 2004 046 167.8 beschrieben.

Dabei kann das eingesetzte Gasgemisch enthaltend Distickstoffmonoxid grundsätzlich aus jeder beliebigen Quelle stammen. Insbesondere ist es möglich, dass als Distickstoffmonoxidquelle das Abgas eines industriellen Verfahrens eingesetzt wird.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Gemäß einer weiteren Ausführungsform betrifft die vorliegende Erfindung daher ein Verfahren zur Herstellung eines Ketons wie oben beschrieben, wobei als Distickstoffmonoxidquelle das Abgas eines industriellen Verfahrens eingesetzt wird.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage einer Glyoxalanlage und/oder einer Salpetersäureanlage, wobei letztere wiederum bevorzugt mit mindestens einem Abgas einer Adipinsäureanlage, einer Dodecandisäureanlage, einer Glyoxalanlage oder einer Hydroxylaminanlage betrieben wird.

Erfindungsgemäß kann das Gasgemisch gasförmig eingesetzt werden. Es ist jedoch auch möglich, das Gasgemisch enthaltend Distickstoffmonoxid zunächst derart zu behandeln, dass das Gasgemisch in flüssiger oder überkritischer Form vorliegt und dann eingesetzt wird. Das Gasgemisch bzw. Distickstoffmonoxid kann durch geeignete Wahl des Drucks oder der Temperatur verflüssigt werden. Ebenso ist es im Rahmen der vorliegenden Erfindung möglich, das Gasgemisch in ein Lösungsmittel einzulösen.

Erfindungsgemäß können die Reaktionsbedingungen bei der erfindungsgemäßen Umsetzung der Zusammensetzung (I) mindestens enthaltend ein 1,1-disubstituiertes Olefin mit der Zusammensetzung enthaltend Distickstoffmonoxid in weiten Bereichen variieren.

Die Temperaturen bei der erfindungsgemäßen Umsetzung der Zusammensetzung (I) mindestens enthaltend ein 1,1-disubstituiertes Olefin mit der Zusammensetzung enthaltend Distickstoffmonoxid liegen bevorzugt im Bereich von 140 bis 350°C, weiter bevorzugt im Bereich von 180 bis 320°C und besonders bevorzugt im Bereich von 200 bis 300°C.

Gemäß einer weiteren Ausführungsform betrifft die vorliegende Erfindung daher ein Verfahren zur Herstellung eines Ketons wie oben beschrieben, wobei die Umsetzung einer Zusammensetzung (I) mindestens enthaltend ein 1,1-disubstituiertes Olefin mit einer Zusammensetzung enthaltend Distickstoffmonoxid bei einer Temperatur im Bereich von 140 bis 350°C durchgeführt wird.

Es ist möglich, die Umsetzung der Zusammensetzung (I) mindestens enthaltend ein 1,1-disubstituiertes Olefin mit der Zusammensetzung enthaltend Distickstoffmonoxid bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der erfindungsgemäßen Umsetzung der Zusammensetzung (I) mindestens enthaltend ein 1,1-disubstituiertes Olefin mit der Zusammensetzung enthaltend Distickstoffmonoxid liegen bevorzugt höher als der Eigendruck des Edukt- bzw. Produktgemisches bei der gewählten Reaktionstemperatur oder den gewählten Reaktionstemperaturen. Die Drücke liegen bevorzugt im Bereich von 1 bis 1000 bar, weiter bevorzugt im Bereich von 40 bis 325 bar und besonders bevorzugt im Bereich von 50 bis 200 bar.

Gemäß einer weiteren Ausführungsform betrifft die vorliegende Erfindung daher ein Verfahren zur Herstellung eines Ketons wie oben beschrieben, wobei die Umsetzung einer Zusammensetzung (I) mindestens enthaltend ein 1,1-disubstituiertes Olefin mit einer Zusammensetzung enthaltend Distickstoffmonoxid bei einem Druck im Bereich von 1 bis 1000 bar durchgeführt wird.

Es ist möglich, die Umsetzung der Zusammensetzung (I) mindestens enthaltend ein 1,1-disubstituiertes Olefin mit der Zusammensetzung enthaltend Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Hinsichtlich der für die Umsetzung der Zusammensetzung (I) mindestens enthaltend ein 1,1-disubstituiertes Olefin mit der Zusammensetzung enthaltend Distickstoffmonoxid einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens einem Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Bevorzugt wird die Umsetzung der Zusammensetzung (I) mindestens enthaltend ein 1,1-disubstituiertes Olefin mit der Zusammensetzung enthaltend Distickstoffmonoxid in einem einzigen Reaktor durchgeführt. Beispielsweise bevorzugt ist die Umsetzung in kontinuierlicher Fahrweise.

Die Verweilzeit des Reaktionsgutes in dem mindestens einen Reaktor bei der Umsetzung des 1,1-disubstituierten Olefins mit Distickstoffmonoxid liegt im allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Im Feed, der der Umsetzung von Distickstoffmonoxid mit dem 1,1-disubstituierten Olefin zugeführt wird, liegt das Molverhältnis von Distickstoffmonoxid zu dem 1,1-disubstituierten Olefin im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Das erfindungsgemäße Verfahren kann darüber hinaus weitere Schritte umfassen. Weitere Schritte können beispielsweise Reinigungsschritte sein. Geeignete Reinigungsschritte sind beispielsweise eine destillative Trennung, insbesondere eine Flash-Destillation. Erfindungsgemäß ist es auch möglich, dass das Verfahren mehrere destillative Trennschritte umfasst, um nicht umgesetzte Edukte oder Lösungsmittel vom gewünschten Produkt abzutrennen. Nicht umgesetzte Edukte und/oder abgetrenntes Lösungsmittel können vorzugsweise in das erfindungsgemäße Verfahren zurückgeführt werden.

Gemäß einer weiteren Ausführungsform betrifft die vorliegende Erfindung daher ein Verfahren zur Herstellung eines Ketons wie oben beschrieben, wobei das Verfahren nach der Umsetzung der Zusammensetzung (I) mindestens enthaltend ein 1,1-disubstituiertes Olefin mit der Zusammensetzung enthaltend Distickstoffmonoxid einen weiteren Reinigungsschritt aufweist.

Mit dem erfindungsgemäßen Verfahren können Ketone, die auf anderen Wegen nur sehr schwer zugänglich sind, in einer einfachen einstufigen Synthese hergestellt werden. Solche Ketone können als beispielsweise als Synthesebausteine verwendet werden oder in anderen Anwendungen wie z.B. als Speziallösemittel, Benzin- oder Dieseladditive, etc. eingesetzt werden.

Im Folgenden wird die vorliegende Erfindung anhand von Beispielen naher erläutert.

### Beispiele

### Beispiel 1 (Vergleichsbeispiel)

Oxidation von 2,2,6,6-Tetramethyl-4-methylenheptan (Triisobutylen) ohne Lösungsmittel bei 260 °C.

Triisobutylen (100 g) wurde in einen 300 mL-Autoklaven eingefüllt, der Autoklav verschlossen und mit N₂ inertisiert. Triisobutylen wurde als technisches Gemisch der Fa. Ineos Innovene (Köln) eingesetzt. Die Hauptkomponenten sind (E/Z)-2,2,4,6,6-Pentamethyl-hept-3-en (∼48%) und 2,2,6,6-Tetramethyl-4-methylene-heptan (∼34%), neben anderen C₁₂H₂₄-Kohlenwasserstoffen (in Summe ∼13%) und geringen Anteilen an Oxygenaten. Der Autoklav wurde dreimal mit 50 bar N₂ gespült und anschließend wird mit N₂O auf 50 bar Kaltdruck aufgepresst. Der Rührer wurde eingeschaltet, auf 400 U/min eingestellt und anschließend auf Solltemperatur (260 °C) erwärmt. Nach 24 h wurde der Autoklav auf Raumtemperatur abgekühlt, auf Umgebungsdruck entspannt und das Produkt per GC analysiert.

Als Austrag wurden erhalten: (E/Z)-2,2,4,6,6-Pentamethyl-hept-3-en (∼35,3 %) und 2,2,6,6-Tetramethyl-4-methylene-heptan (30,5 %), 4,4-Dimethyl-pentan-2-on (4,3 %), Dineopentylketon (4,0 %), 2-*tert*-Butyl-1-(2,2-dimethyl-propyl)-1-methyl-cyclopropan (1,6 %) sowie weitere Verbindungen.

Das Verhältnis zwischen Dineopentylketon und das Cyclopropanderivat betrug in diesem Fall 2,5 g/g.

### Beispiel 2 (Vergleichsbeispiel)

Oxidation von 2,2,6,6-Tetramethyl-4-methylenheptan (Triisobutylen) in nicht erfindungsgemäßem Lösungsmittel (Cyclohexan) bei 260 °C.

Triisobutylen (10 g) und Cyclohexan (90 g) wurde in einen 300mL-Autoklaven eingefüllt, der Autoklav verschlossen und mit N₂ inertisiert. Triisobutylen wurde als technisches Gemisch der Fa. Ineos Innovene (Köln) eingesetzt. Die Hauptkomponenten sind (E/Z)-2,2,4,6,6-Pentamethyl-hept-3-en (∼48%) und 2,2,6,6-Tetramethyl-4-methyleneheptan (∼34%), neben anderen C₁₂H₂₄-Kohlenwasserstoffen (in Summe ∼13%) und geringen Anteilen an Oxygenaten. Der Autoklav wurde dreimal mit 50 bar N₂ gespült und anschließend wird mit N₂O ohne Rührerbewegung auf 50 bar Kaltdruck aufgepresst. Der Rührer wurde eingeschaltet, auf 400 U/min eingestellt und anschließend auf Solltemperatur (260 °C) erwärmt. Nach 24 h wurde der Autoklav auf Raumtemperatur abgekühlt, auf Umgebungsdruck entspannt und das Produkt per GC analysiert.

Als Austrag wurden erhalten (ohne Cyclohexan): (E/Z)-2,2,4,6,6-Pentamethyl-hept-3-en (∼29,7 %) und 2,2,6,6-Tetramethyl-4-methylene-heptan (24,5 %), 4,4-Dimethylpentan-2-on (3,2 %), Dineopentylketon (3,2 %), 2-*tert*-Butyl-1-(2,2-dimethyl-propyl)-1-methyl-cyclopropan (0,3 %) sowie weitere Verbindungen.

Das Verhältnis zwischen Dineopentylketon und das Cyclopropanderivat betrug in diesem Fall 10,7 g/g. Dies Ergebnis ist besser als das gemäß Beispiel 1 erhaltene, aber immer noch unbefriedigend.

### Beispiel 3

Oxidation von 2,2,6,6-Tetramethyl-4-methylenheptan (Triisobutylen) mit erfindungsgemäßem Lösungsmittel (Methanol) bei 260 °C.

Triisobutylen (10 g) und Methanol (90 g) wurden in einen 300mL-Autoklaven eingefüllt, der Autoklav verschlossen und mit N₂ inertisiert. Triisobutylen wurde als technisches Gemisch der Fa. Ineos Innovene (Köln) eingesetzt. Die Hauptkomponenten sind (E/Z)-2,2,4,6,6-Pentamethyl-hept-3-en (∼48%) und 2,2,6,6-Tetramethyl-4-methylene-heptan (∼34%), neben anderen C₁₂H₂₄-Kohlenwasserstoffen (in Summe ∼13%) und geringen Anteilen an Oxygenaten. Der Autoklav wurde dreimal mit 50 bar N₂ gespült und anschließend wird mit N₂O ohne 50 bar Kaltdruck aufgepresst. Der Rührer wurde eingeschaltet, auf 400 U/min eingestellt und anschließend auf Solltemperatur (260 °C) erwärmt. Nach 24 h wurde der Autoklav auf Raumtemperatur abgekühlt, auf Umgebungsdruck entspannt und das Produkt per GC analysiert.

Als Austrag wurden erhalten (ohne Methanol): (E/Z)-2,2,4,6,6-Pentamethyl-hept-3-en (∼45,3 %) und 2,2,6,6-Tetramethyl-4-methylene-heptan (34,2 %), 4,4-Dimethyl-pentan-2-on (2,0 %), Dineopentylketon (3,6 %), 2-*tert*-Butyl-1-(2,2-dimethyl-propyl)-1-methyl-cyclopropan (0,2 %) sowie weitere Verbindungen.

Das Verhältnis zwischen Dineopentylketon und das Cyclopropanderivat betrug in diesem Fall 18 g/g und war damit deutlich höher als gemäß Beispiel 1 und 2.

### Beispiel 4 (Vergleichsbeispiel)

Oxidation von 2,2,6,6-Tetramethyl-4-methylenheptan (Triisobutylen) ohne Lösungsmittel bei 290 °C (erhöhte Temperatur).

Triisobutylen (100 g) wurde in einen 300mL-Autoklaven eingefüllt, der Autoklav verschlossen und mit N₂ inertisiert. Triisobutylen wurde als technisches Gemisch der Fa. Ineos Innovene (Köln) eingesetzt. Die Hauptkomponenten sind (E/Z)-2,2,4,6,6-Pentamethyl-hept-3-en (∼48%) und 2,2,6,6-Tetramethyl-4-methylene-heptan (∼34%), neben anderen C₁₂H₂₄-Kohlenwasserstoffen (in Summe ∼13%) und geringen Anteilen an Oxygenaten. Der Autoklav wurde dreimal mit 50 bar N₂ gespült und anschließend wird mit N₂O ohne Rührerbewegung auf 50 bar Kaltdruck aufgepresst. Der Rührer wurde eingeschaltet, auf 400 U/min eingestellt und anschließend auf Solltemperatur (290 °C) erwärmt. Nach 24 h wurde der Autoklav auf Raumtemperatur abgekühlt, auf Umgebungsdruck entspannt und das Produkt per GC analysiert.

Austrag: (E/Z)-2,2,4,6,6-Pentamethyl-hept-3-en (32,2 %) und 2,2,6,6-Tetramethyl-4-methylene-heptan (30,4 %), 4,4-Dimethyl-pentan-2-on (7,12 %), Dineopentylketon (5,9 %), 2-*tert*-Butyl-1-(2,2-dimethyl-propyl)-1-methyl-cyclopropan (2,6 %) sowie weitere Verbindungen.

Das Verhältnis zwischen Dineopentylketon und das Cyclopropanderivat betrug in diesem Fall 2,26 g/g.

### Beispiel 5

Oxidation von 2,2,6,6-Tetramethyl-4-methylenheptan (Triisobutylen) mit erfindungsgemäßem Lösungsmittel (Methanol) bei 290 °C (erhöhte Temperatur).

Triisobutylen (10 g) und Methanol (90 g) wurden in einen 300mL-Autoklaven eingefüllt, der Autoklav verschlossen und mit N₂ inertisiert. Triisobutylen wurde als technisches Gemisch der Fa. Ineos Innovene (Köln) eingesetzt. Die Hauptkomponenten sind (E/Z)-2,2,4,6,6-Pentamethyl-hept-3-en (∼48%) und 2,2,6,6-Tetramethyl-4-methylene-heptan (∼34%), neben anderen C₁₂H₂₄-Kohlenwasserstoffen (in Summe ∼13%) und geringen Anteilen an Oxygenaten. Der Autoklav wurde dreimal mit 50 bar N₂ gespült und anschließend wird mit N₂O ohne Rührerbewegung auf 50 bar Kaltdruck aufgepresst. Der Rührer wurde eingeschaltet, auf 400 U/min eingestellt und anschließend auf Solltemperatur (290 °C) erwärmt. Nach 24 h wurde der Autoklav auf Raumtemperatur abgekühlt, auf Umgebungsdruck entspannt und das Produkt per GC analysiert.

Austrag (ohne Methanol): (E/Z)-2,2,4,6,6-Pentamethyl-hept-3-en (15,3 %) und 2,2,6,6-Tetramethyl-4-methylene-heptan (28,4 %), 4,4-Dimethyl-pentan-2-on (10,0 %), Dineopentylketon (16,1 %), 2-*tert-*Butyl-1-(2,2-dimethyl-propyl)-1-methyl-cyclopropan (0,2 %) sowie weitere Verbindungen.

Das Verhältnis zwischen Dineopentylketon und das Cyclopropanderivat betrug in diesem Fall 80,5 g/g.

### Beispiel 6 (Vergleichsbeispiel)

### Oxidation von 2,4,4-Trimethyl-1-penten (α-Diisobutylen) ohne Lösungsmittel

alpha-Diisobutylen (100 g) wurde in einen 300mL-Autoklaven eingefüllt, der Autoklav verschlossen und mit N₂ inertisiert. Der Autoklav wurde dreimal mit 50 bar N₂ gespült und anschließend wird mit N₂O auf 50 bar Kaltdruck aufgepresst. Der Rührer wurde eingeschaltet, auf 400 U/min eingestellt und anschließend auf Solltemperatur (290 °C) erwärmt. Nach 24 h wurde der Autoklav auf Raumtemperatur abgekühlt, auf Umgebungsdruck entspannt und das Produkt per GC analysiert.

Austrag (ohne Methanol): Aceton (0,5 %), alpha-Diisobutylen (26,4 %), Methylneopentyl-keton (25,1 %), 2-*tert*-Butyl-1,1-dimethyl-cyclopropan (10,4 %) sowie weitere Verbindungen.

Das Verhältnis zwischen Methylneopentylketon und das Cyclopropanderivat betrug 2,54 g/g.

### Beispiel 7

### Oxidation von 2,4,4-Trimethyl-1-penten (alpha-Diisobutylen) mit erfindungsgemäßen Lösungsmittel (Methanol)

alpha-Diisobutylen (10 g) und Methanol (90 g) wurden in einen 300mL-Autoklaven eingefüllt, der Autoklav verschlossen und mit N₂ inertisiert. Der Autoklav wurde dreimal mit 50 bar N₂ gespült und anschließend wird mit N₂O auf 50 bar Kaltdruck aufgepresst. Der Rührer wurde eingeschaltet, auf 400 U/min eingestellt und anschließend auf Solltemperatur (290 °C) erwärmt. Nach 24 h wurde der Autoklav auf Raumtemperatur abgekühlt, auf Umgebungsdruck entspannt und das Produkt per GC analysiert.

Austrag (ohne Methanol): Aceton (1,2 %), alpha-Diisobutylen (21,9 %), Methylneopentyl-keton (32 %), 2-*tert*-Butyl-1,1-dimethyl-cyclopropan (0,4 %) sowie weitere Verbindungen.

Verhältnis zwischen Methylneopentylketon und das Cyclopropanderivat betrug in diesem Fall 80 g/g.

## Patentansprüche

1. Verfahren zur Herstellung eines Ketons der allgemeinen Formel (1) 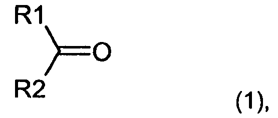 worin R1 und R2 unabhängig voneinander ausgewählt sind aus substituierten und unsubstituierten Alkylresten mit 1 bis 20 Kohlenstoffatomen, und substituierten und unsubstituierten Arylresten, und wobei die Reste R1 und R2 auch miteinander verbunden sein und einen Ring bilden können,
umfassend die Umsetzung einer Zusammensetzung (I) mindestens enthaltend ein 1,1-disubstituiertes Olefin der allgemeinen Formel (2) 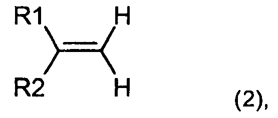 mit einer Zusammensetzung enthaltend Distickstoffmonoxid, wobei die Umsetzung in Gegenwart eines Lösungsmittels erfolgt, das mindestens eine protonenliefernde funktionelle Gruppe enthält und aus der Gruppe der Lösungsmittel, die mindestens eine COOH-Gruppe aufweisen, der Lösungsmittel, die mindestens eine OH-Gruppe aufweisen, und der Lösungsmittel, die mindestens eine NH-Gruppe aufweisen, ausgewählt ist.

2. Verfahren zur Herstellung eines Ketons nach Anspruch 1, wobei das Lösungsmittel ausgewählt ist aus der Gruppe bestehend aus Carbonsäuren, Aminosäuren, Wasser, Alkoholen, Ammoniak, Hydrazin, Aminen und Amiden.

3. Verfahren zur Herstellung eines Ketons nach Anspruch 1 oder 2, wobei die Reste R1 und R2 ausgewählt sind aus substituierten und unsubstituierten Alkylresten mit 1 bis 6 Kohlenstoffatomen und substituierten und unsubstituierten Arylresten.

4. Verfahren zur Herstellung eines Ketons nach einem der Ansprüche 1 bis 3, wobei das 1,1-disubstituierte Olefin ausgewählt ist aus der Gruppe bestehend aus Isobuten, Methylencyclopropan, 2-Methyl-1-buten, 2,4,4-Trimethyl-1-penten, 2,2,6,6-Tetramethyl-4-methylen-heptan, alpha-Polyisobuten, Methylencyclopentan, Methylencyclohexan, beta-Pinen und 1,1-Diphenylethen.

5. Verfahren zur Herstellung eines Ketons nach einem der Ansprüche 1 bis 4, wobei als Distickstoffmonoxidquelle das Abgas eines industriellen Verfahrens eingesetzt wird.

6. Verfahren zur Herstellung eines Ketons nach einem der Ansprüche 1 bis 5, wobei die Umsetzung der Zusammensetzung (I) mindestens enthaltend ein 1,1-disubstituiertes Olefin mit der Zusammensetzung enthaltend Distickstoffmonoxid bei einem Druck im Bereich von 1 bis 1000 bar durchgeführt wird.

7. Verfahren zur Herstellung eines Ketons nach einem der Ansprüche 1 bis 6, wobei die Umsetzung der Zusammensetzung (I) mindestens enthaltend ein 1,1-disubstituiertes Olefin mit der Zusammensetzung enthaltend Distickstoffmonoxid bei einer Temperatur im Bereich von 140 bis 350°C durchgeführt wird.

8. Verfahren zur Herstellung eines Ketons nach einem der Ansprüche 1 bis 7, wobei das Verfahren nach der Umsetzung der Zusammensetzung (I) mindestens enthaltend ein 1,1-disubstituiertes Olefin mit der Zusammensetzung enthaltend Distickstoffmonoxid einen weiteren Reinigungsschritt aufweist.

## Claims

1. A process for preparing a ketone of the general formula (1) 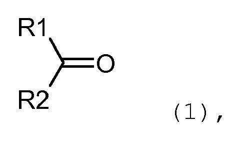 in which R1 and R2 are each independently selected from substituted and unsubstituted alkyl radicals having 1 to 20 carbon atoms, and substituted and unsubstituted aryl radicals, and where the R1 and R2 radicals may also be joined to one another and form a ring,
comprising the reaction of a composition (I) at least comprising a 1,1-disubstituted olefin of the general formula (2) 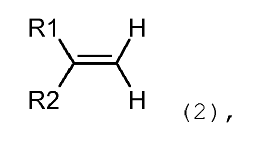 with a composition comprising dinitrogen monoxide, wherein the reaction is effected in the presence of a solvent which comprises at least one proton-donating functional group and is selected from the group of the solvents which have at least one COOH group, the solvents which have at least one OH group, and the solvents which have at least one NH group.

2. The process for preparing a ketone according to claim 1, wherein the solvent is selected from the group consisting of carboxylic acids, amino acids, water, alcohols, ammonia, hydrazine, amines and amides.

3. The process for preparing a ketone according to claim 1 or 2, wherein the R1 and R2 radicals are selected from substituted and unsubstituted alkyl radicals having 1 to 6 carbon atoms and substituted and unsubstituted aryl radicals.

4. The process for preparing a ketone according to any one of claims 1 to 3, wherein the 1,1-disubstituted olefin is selected from the group consisting of isobutene, methylenecyclopropane, 2-methyl-1-butene, 2,4,4-trimethyl-1-pentene, 2,2,6,6-tetramethyl-4-methyleneheptane, alpha-polyisobutene, methylenecyclopentane, methylenecyclohexane, beta-pinene and 1,1-diphenylethene.

5. The process for preparing a ketone according to any one of claims 1 to 4, wherein the dinitrogen monoxide source used is the offgas of an industrial process.

6. The process for preparing a ketone according to any one of claims 1 to 5, wherein the reaction of the composition (I) at least comprising a 1,1-disubstituted olefin with the composition comprising dinitrogen monoxide is performed at a pressure in the range from 1 to 1000 bar.

7. The process for preparing a ketone according to any one of claims 1 to 6, wherein the reaction of the composition (I) at least comprising a 1,1-disubstituted olefin with the composition comprising dinitrogen monoxide is performed at a temperature in the range from 140 to 350°C.

8. The process for preparing a ketone according to any one of claims 1 to 7, wherein the process, after the reaction of the composition (I) at least comprising a 1,1-disubstituted olefin with the composition comprising dinitrogen monoxide, has a further purification step.

## Revendications

1. Procédé pour la préparation d'une cétone de formule générale (1) 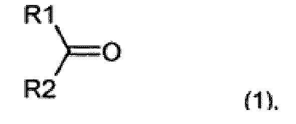 où R1 et R2 sont choisis, indépendamment l'un de l'autre, parmi les radicaux alkyle substitués et non substitués comprenant 1 à 20 atomes de carbone et les radicaux aryle substitués et non substitués et les radicaux R1 et R2 pouvant également être reliés l'un à l'autre et former un cycle,
comprenant la transformation d'une composition (I) contenant au moins une oléfine 1,1-disubstituée de formule générale (2) 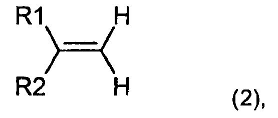 avec une composition contenant du protoxyde d'azote, la transformation ayant lieu en présence d'un solvant qui contient au moins un groupe fonctionnel fournissant des protons et choisi dans le groupe des solvants qui présentent au moins un groupe COOH, des solvants qui présentent au moins un groupe OH et des solvants qui présentent au moins un groupe NH.

2. Procédé pour la préparation d'une cétone selon la revendication 1, le solvant étant choisi dans le groupe constitué par les acides carboxyliques, les aminoacides, l'eau, les alcools, l'ammoniaque, l'hydrazine, les amines et les amides.

3. Procédé pour la préparation d'une cétone selon la revendication 1 ou 2, les radicaux R1 et R2 étant choisis parmi les radicaux alkyle substitués et non substitués comprenant 1 à 6 atomes de carbone et les radicaux aryle substitués et non substitués.

4. Procédé pour la préparation d'une cétone selon l'une quelconque des revendications 1 à 3, l'oléfine 1,1-disubstituée étant choisie dans le groupe constitué par l'isobutène, le méthylènecyclopropane, le 2-méthyl-1-butène, le 2,4,4-triméthyl-1-pentène, le 2,2,6,6-tétraméthyl-4-méthylèneheptane, l'alpha-polyisobutène, le méthylènecyclopentane, le méthylènecyclohexane, le bêta-pinène et le 1,1-diphényléthène.

5. Procédé pour la préparation d'une cétone selon l'une quelconque des revendications 1 à 4, en utilisant, comme source de protoxyde d'azote, l'effluent gazeux d'un procédé industriel.

6. Procédé pour la préparation d'une cétone selon l'une quelconque des revendications 1 à 5, la transformation de la composition (I) contenant au moins une oléfine 1,1-disubstituée avec la composition contenant du protoxyde d'azote étant réalisée à une pression dans la plage de 1 à 1000 bars.

7. Procédé pour la préparation d'une cétone selon l'une quelconque des revendications 1 à 6, la transformation de la composition (I) contenant au moins une oléfine 1,1-disubstituée avec la composition contenant du protoxyde d'azote étant réalisée à une température dans la plage de 140 à 350°C.

8. Procédé pour la préparation d'une cétone selon l'une quelconque des revendications 1 à 7, le procédé présentant, après la transformation de la composition (I) contenant au moins une oléfine 1,1-disubstituée avec la composition contenant du protoxyde d'azote, une autre étape de purification.
